Europäisches Patentamt

European Patent Office

Office européen des brevets

(19)

(11) Publication number: **0 132 754 B1**

## (12) EUROPEAN PATENT SPECIFICATION

(45) Date of publication of patent specification: **27.03.91**

(51) Int. Cl.⁵: **A61K 37/66,** A61K 37/02, G01N 33/50

(21) Application number: **84108378.5**

(22) Date of filing: **17.07.84**

(54) **Use of gamma-interferon in the manufacture of a medicament for the treatment of leprosy.**

(30) Priority: **29.07.83 US 518552**

(43) Date of publication of application:
**13.02.85 Bulletin 85/07**

(45) Publication of the grant of the patent:
**27.03.91 Bulletin 91/13**

(84) Designated Contracting States:
**DE FR GB**

(56) References cited:
**WO-A-83/01198**

**CHEMICAL ABSTRACTS, vol. 100, no. 9, 27th February 1984, page 453, abstract no. 66392u, Columbus, Ohio, US; N. NOGUEIRA et al.: "Defective gamma interferon production in leprosy. Reversal with antigen and interleukin 2." & J.EXP.MED. 1983, 158(6), 2165-70**

(73) Proprietor: **THE ROCKEFELLER UNIVERSITY**
**1230 York Avenue**
**New York, NY 10021(US)**

(72) Inventor: **Nogueira, Nadia**
**6 Greenholm**
**Princeton New Jersey 08540(US)**
Inventor: **Steinman, Ralph**
**62 North Avenue**
**Westport Connecticut 06880(US)**

(74) Representative: **Schulze Horn, Stefan, Dipl.-Ing. M.Sc.**
**Goldstrasse 36**
**W-4400 Münster(DE)**

**Description**

Leprosy is a chronic disease caused by Mycobacteria leprae. In some subjects, the disease is disseminated (lepromatous form). In these, widespread lesions present lose infiltrates and the organisms are found in large numbers in skin macrophages. Such individuals have a predominant role in transmitting the disease. The rapid progression of this type of lesion and their heavy bacillary load has been ascribed to deficient T-cell response to the specific antigen, M. leprae, but not to other antigens (Turk, J.L. and Bryuson, A.D.M. Adv. Immunol. 13 : 209-266 (1971); Myrvang, B., Godal, T., Ridley, D.S., Froland, S.S., and Song, Y.K. Clin. Exp. Immun. 14 : 541-553 (1973)). In contrast, subjects with the less severe form of the disease (tuberculoid) develop strong cell-mediated immunity that results in effective killing of bacteria inside macrophages. T-cells from these patients respond strongly to M. leprae antigen.

Studies of the lesions of leprosy subjects have revealed that in the lepromatous cases of leprosy the cellular infiltrates are devoid of "helper" T-cells, as identi fied by OKT4/Leu 3a monoclonal antibodies. This is in contrast to tuberculoid patients where a predominance of "helper" T-cells is found (Van Voorhis, W.C., Kaplan G., Sarno, E.N., Horwitz, M.A., Steinman, R.M., Lewis, W.R., Nogueira, N., Hair, L.S., Gattass, C.R., Arrick, B.A. and Cohn, Z.A. New England J. Medicine 307 : 1593-1597 (1982).

In the process of macrophage activation, helper-type T-cells generate factors ("lymphokines") which lead to functional changes in macrophage function leading to a microbicidal state (Nogueira, N., Gordon, S. and Cohn Z. J. Exp. Med. 146 : 172 1977; Nogueira, N. and Cohn, Z.A. J. Exp. Med. 148 : 288 1978; Nogueira, N., Chaplan, S. Reesink, M., Tydings J. and Cohn, Z.A. J. Immunol. 128 : 2142 1982). Among other soluble factors, $\gamma$-interferon seems to be a large component of such "lymphokine" preparations (waksman, B.H. Biology of the Lmphokines S. Cohen, E. Pck and J.J. Oppenheim, editors Academic Press, New York 585-616 1979). $\gamma$-interferon has been shown in a tumor cell mode to copurify with a "macrophage activating factor" (Pace, J.L., Russel, S.W., Schneider, R.D., Altman, A. and Katz, D.H. P.N.A.S. 3782-3786 1983; Wisseman, C.L. Jr. and Waddell, A. J. Exp. Med. 1780-1993 1983): One Document, WO 830 1198 discloses the use of $\gamma$-interferon in combination with antibiotics for the treatment of bacterially/fungally contaminated/infected areas.

Accordingly, the role of $\gamma$-interferon in the progress of leprosy has been studied. Specifically, peripheral blood mononuclear cells from patients with both lepromatous and benign tuberculoid leprosy were examined, in the presence of M. leprae antigen and mitrogens such as Conconavalin A, in terms of capacity for $\gamma$-interferon release.

Patients with leprosy were examined for the capacity of their peripheral blood mononuclear cells to respond to M . leprae antigen and to mitogens, such as Concanavalin A in terms of $\gamma$-interferon release. $\gamma$-interferon was measured by anti-viral activity in human foreskin cells infected with encephalo-myocarditis (EMC) virus; $\gamma$-interferon was identified by the use of a $\gamma$ - specific rabbit antibody which specifically neutralized $\gamma$-interferon activity.

Table I

-IFN Release by RBMC of Leprosy Patients

| Patients | Treatment | Clinical Diagnosis | Hist. Diagnosis | -IFN (U/ml) | |
|---|---|---|---|---|---|
| | | | | M. Leprae | Con A |
| SRG | None | L | LL | 0 | 0 |
| EAS | None | L | LL | 0 | 0 |
| AA | 1 yr. | L | LL | 0 | 0 |
| RM | 3 yr. | L | LL | 16 | 32 |
| FEG | 1 yr. | L | LL | 8 | 8 |
| CL | 2 yr. | L | LL | 0 | 16 |
| APA | None | L | LL | 0 | 0 |
| AL | 1 yr. | L | LL | 0 | 0 |
| CBS | None | L | BL | 0 | 0 |
| SS | | T | BB | 64 | 16 |
| ML | | T | BT | 32 | 64 |
| RC MP | | B | BT | 128 | 512 |
| RS | | T | BT | 128 | 128 |
| MV | | T | BT | 256 | ND |
| BEBS | | T | TT | 256 | 256 |

## Claims

1. Use of γ -interferon for the preparation of a therapeutic drug not containing antibiotics, for the treatment of leprosy.

## Revendications

1. Utilisation du γ -interféron pour la préparation d'un médicament thérapeutique, ne contenant pas d'antibiotiques, pour le traitement de la lèpre.

## Ansprüche

1. Die Verwendung von Gamma-Interferon für die Herstellung eines für die Behandlung von Lepra einzusetzenden Arznei-Mittels, das keine Antibiotika enhält.